(19) Europäisches Patentamt / European Patent Office / Office européen des brevets

(11) **EP 2 902 008 B1**

(12) **EUROPEAN PATENT SPECIFICATION**

(45) Date of publication and mention
of the grant of the patent:
**13.02.2019 Bulletin 2019/07**

(21) Application number: **13830900.0**

(22) Date of filing: **19.08.2013**

(51) Int Cl.:
*A61K 8/24* *(2006.01)*        *A61K 8/66* *(2006.01)*
*A61K 8/9789* *(2017.01)*       *A61Q 11/00* *(2006.01)*
*A61Q 11/02* *(2006.01)*

(86) International application number:
**PCT/RU2013/000716**

(87) International publication number:
**WO 2014/031035 (27.02.2014 Gazette 2014/09)**

(54) **MINERAL-ENZYME COMPLEX FOR FORTIFYING AND WHITENING TOOTH ENAMEL, ORAL HYGIENE COMPOSITION AND TOOTHPASTE**

MINERALENZYMKOMPLEXE ZUR STÄRKUNG UND AUFHELLUNG DES ZAHNSCHMELZES, MUNDHYGIENEMITTEL UND ZAHNPASTA

COMPLEXE DE MINÉRAUX ET FERMENTS POUR LE RENFORCEMENT ET LE BLANCHISSEMENT DE L'ÉMAIL DENTAIRE, COMPOSITION POUR L'HYGIÈNE BUCCALE ET PÂTE DENTIFRICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2012 RU 2012135577**

(43) Date of publication of application:
**05.08.2015 Bulletin 2015/32**

(73) Proprietor: **LIMITED LIABILITY COMPANY SPLAT GLOBAL**
**Novgorod Region 174350 (RU)**

(72) Inventors:
• **BELOUS, Elena Yurievna**
  **Moscow 121309 (RU)**
• **MALTABAR, Svetlana Alekseevna**
  **Moskovskaya oblats**
  **g. Odintsovo 143005 (RU)**

• **GALIMOVA, Anna Zufarovna**
  **Moskovskaya obl.**
  **g. Aprelevka 121099 (RU)**

(74) Representative: **Glawe, Delfs, Moll Partnerschaft mbB von Patent- und Rechtsanwälten**
**Postfach 13 03 91**
**20103 Hamburg (DE)**

(56) References cited:
EP-A1- 2 095 724        WO-A1-01/64175
FR-A1- 2 617 710        JP-A- S62 249 917
JP-A- 2007 308 429      RU-C1- 2 131 723
RU-C2- 2 381 021        US-A- 3 802 997
US-A1- 2010 129 297

• LEONTIEV A.A. ET AL.: 'Klinicheskie issledovaniya antisensitivnoi zubnoi pasty ''Asepta Sensitin''.' PARODONTOLOGIYA vol. 2, no. 51, 2009, pages 61 - 63, XP008179212

**Description**

**Field of invention.**

This invention relates to cosmetology, specifically, to a mineral-enzyme complex for strengthening and whitening tooth enamel, and also to compositions for oral cavity hygiene comprising such complex, in particular, toothpastes and other compositions.

**Prior art.**

Patent literature comprises many publications disclosing the use of both individual minerals and mineral complexes in a variety of compositions for oral cavity hygiene, in particular, comprising calcium hydroxyapatite (calcium hydroxyphosphate).

Patent RU 2355380 claiming "A tooth elixir for the prevention and treatment of initial dental caries forms" discloses a mineral complex comprising calcium hydroxyapatite - a mineralizor contributing to the changing of tooth enamel mineralization kinetics, and sodium fluoride preventing the adsorption of organic substances and microorganisms on the surface of the enamel and participating in enamel prism formation.

A complex is also known comprising calcium hydroxyapatite and potassium citrate. Potassium citrate reduces the response of nerve fibres in exposed dental tubules to temperature, osmotic and tectile irritants. Calcium hydroxyapatite, in turn, fills dental tubules from the entrance aperture to the depth of the tubule, tightly sealing the entrance itself, which terminates the liquor outflow from dental tubules to the outside. As a result of that, intra-tubular osmotic pressure is restored, and pain syndrome in respect of external irritants is significantly reduced. The complex is designed to improve oral hygiene agent desensitizing action (see A.A. LEONTIEV et al. "Clinical Research of 'Asepta Sensitive' Anti-Sensitive Toothpaste", Stomatology Today, No.7 (87), 2009).

The same source also describes toothpaste, which, beside the described complex, also comprises thermal mud, marigold, melilot and calamus extracts, xylitol and papain enzyme, which taken in a complex, allow the paste to render not just desensitizing, but also anti-inflammation and anti-plaque action.

The use of mineral complexes in oral cavity hygiene compositions allows to improve tooth mineralization, increase enamel resistance etc. The use of calcium hydroxyapatite in a dentifrice composition providing desensitizing and anti-inflammatory effects is also known from RU2131723.

Disadvantages of known complexes include insufficient cleaning properties of the hygienic agents using such complexes, and inadequate tooth whitening.

These deficiencies may be eliminated by using individual enzymes or enzyme complexes in mouth cavity hygiene agents, disclosed quite broadly in the prior art.

Thus, for example, it is known that enzymes in the form of immobilized Bacillus subtilis proteinases, ensure cleaning and anti-inflammation action of mouth cavity hygienic agents (see patent RU2060030), while such enzyme as tannase, when used in oral cavity hygiene agents, helps remove color blemishes since it hydrolizes tannins contained in tea and coffee that cause tooth discoloration, (see specification to patent RU2416391).

Enzymes may be present as a set of substances. Patent RU2355420 discloses a complex of enzyme-active substances comprising papain, lidase, ribonuclease and lysozyme for the purpose of increasing cleaning, anti-microbial and anti-inflammation action, as well as restoration of the natural oral cavity microflora.

Also a toothpaste is disclosed herein using the above complex, comprising the following elements, mass %:

| | |
|---|---|
| silicium dioxide | 10.0-25.0 |
| glycerin | 5.0-10.0 |
| PEG 400 | 2.0-3.0 |
| xylitol | 5.0-6.0 |
| disodium EDTA | 0.2-1.0 |
| papain | 0.2-1.0 |
| lysozyme | 0.01-0.4 |
| lactolose | 2.0-6.0 |
| sodium carboxy-methylcellulose | 0.5-0.7 |
| carbomer | 0.2-0.4 |
| ribonuclease | 0.001-0.05 |
| lidase | 0.005-0.05 |
| sodium lauryl sarcosinate | 0.1-1.0 |

(continued)

| | |
|---|---|
| sodium lauryl sulfate | 0.5-2.0 |
| cocamidopropyl betaine | 0.5-3.0 |
| reduced glutathione | 0.001-0.01 |
| Extracts: camomile, parsley | 0.05-1.0 |
| grapeseed | 0.05-1.0 |
| glucospheres | 0.5-3.0 |
| tetrapotassium pyrophosphate | 2.0-5.0 |
| tetrasodium pyrophosphate | 0.5-2.0 |
| disodium pyrophosphate | 0.3-1.0 |
| sodium saccharinate | 0.1-0.6 |
| citric acid | 0.1-0.4 |
| sodium citrate | 0.1-2.0 |
| fir tree extract | 0.05-1.0 |
| sage extract | 0.05-1.0 |
| chondroitin sulphate | 0.1-0.5 |
| betaine | 2.0-4.0 |
| aminefluoride | 0.19-1.95 |
| sodium fluoride | 0.1-0.3 |
| sodium methyl paraben | 0.1-0.5 |
| sodium propyl paraben | 0.1-0.3 |
| titanium dioxide | 0.3-0.5 |
| flavoring agent | 0.05-2.0 |
| polyvinyl pyrrolidone | 0.1-0.5 |
| demineralized water | 10.84-66.893 |

This complex and toothpaste using the same have whitening and antimicrobial properties, but do not exert a mineralizing effect on the hard tissues of the teeth, strengthen the enamel but weakly, and do not possess sufficient anti-inflammatory action.

As follows from the above sources, calcium hydroxyapatite based complexes provide a solution to the problem of improving dental tissue mineralization, while enzyme based complexes improve teeth whitening, i.e. they are rather narrowly specialized and do not possess the entire range of protective properties. In particular, none of the complexes under study, or any dental hygiene agents containing the same, possesses a combined anti-inflammatory and blood-stopping action along with teeth cleaning and whitening.

**Disclosure of the invention.**

The objective of this invention is to improve consumer properties of the complex and oral cavity hygiene agents using the claimed mineral-enzyme complex, including: cleaning ability, whitening properties, reduction of gum inflammation and bleeding, and improved desensitizing effect, while retaining the remineralizing effect in respect of dental tissues. The set objective is achieved through the use of a mineral-enzyme complex for tooth enamel strengthening and whitening, comprising calcium hydroxyapatite and tannase with the following component proportions: 0.2 to 10 parts of tannase per 100 mass parts of calcium hydroxyapatite.

In particular embodiments of the invention, the set objective is achieved by adding grapeseed extract with the following component proportions: 0.2 to 10 parts of tannase and up to 100 mass parts of grapeseed extract per 100 mass parts of calcium hydroxyapatite.

The set objective is also achieved by means of a composition for oral cavity hygiene comprising the above-said mineral-enzyme complex for tooth enamel strengthening in an effective quantity and an appropriate medium.

In particular embodiments of the invention, the said composition may represent a toothpaste, rinsing composition, foaming rinsing composition or a chewing gum.

The set objective is also achieved by means of a toothpaste for oral cavity hygiene comprising the said mineral-enzyme complex for tooth enamel strengthening in an effective quantity and an appropriate medium, said toothpaste additionally comprising substances selected from a group containing abrasive substances, moisturizers, thickeners, surfactants and solvents.

The toothpaste may additionally comprise substances selected from a group containing colorants, preservatives, flavoring agents, anti-oxidizing agents, mineralizing agents, vitamins and mixtures thereof.

In particular embodiments of the invention, a toothpaste comprises a medium containing water as a solvent, silicium dioxide as an abrasive substance, sodium carboxy-methylcellulose as a thickener, at least one moisturizer selected from a group comprising glycerin, sorbitol and polyethylene glycol, and sodium lauryl sarcosinate as a surfactant, with the following component proportions, mass %:

| | |
|---|---|
| Mineral-enzyme complex | 1.50-30.00 |
| Silicium dioxide | 1.00 - 60.00 |
| Sodium carboxy-methylcellulose | 0.50 - 10.00 |
| Moisturizer | 0.50 - 7000 |
| Sodium lauryl sarcosinate | 0.01 - 10.00 |
| Water | the rest. |

That toothpaste may additionally contain xanthan gum as a thickener in a quantity not exceeding 3 mass %.

The toothpaste may additionally contain substances selected from a group containing foam stabilizers, colorants, preservatives, flavoring agents, anti-oxidizing agents, mineralizing agents, anti-inflammatory binding agents, aseptics, anti-caries agents and mixtures thereof with the following proportions, mass %:

| | |
|---|---|
| Foam stabilizers | max. 5.00 |
| Colorants | max. 5.00 |
| Preservatives | max. 0.30 |
| Flavoring agents | max. 3.00 |
| Anti-oxidizing agents | max. 5.00 |
| Mineralizing agents | max. 10.00 |
| Anti-inflammatory binding agents | max. 10.00 |
| Aseptics | max. 1.00 |
| Anti-caries agents | max. |

The essence of the invention is as follows.

Calcium hydroxyapatite$\tau$ possesses high adhesion and resorption properties in respect of all types of tissue, both bone and epithelial. As a result of resorption, soft tissues develop a protective coating, and the process of regeneration/granulation is promoted with the generation of new cells and tissues at the place of injury or inflammation.

Tannase is an esterase group enzyme. This enzyme catalyzes hydrolitic cleavage of tannine. There is information in literature that tannase is strictly specific in its action: it disintegrates complex ethers whose acidic component contains at least two phenolic hydroxyls.

We assume that tannase is capable of catalyzing processes in the mouth cavity with the production of antibaterial substances that enhance, in a non-specific manner, anti-inflammatory properties of calcium hydroxyapatite, at the same time ensuring intensive teeth whitening. For the claimed complex, it is advisable that amorphous calcium hydroxyapatite with a particle size of 20-80 nm be used. Such calcium hydroxyapatite form does not have abrasive properties while possessing improved biocompatibility with dental tissue.

Research has shown that all the above mineral-enzyme complex components should be gathered together in certain proportions in the complex; in that case, there is a synergetic effect which provides an opportunity to improve tooth enamel and periodontal tissue conditions in a non-liner fashion.

Beyond the claimed proportions of the components, the claimed technical result is not achieved.

The mineral-enzyme complex may contain up to 100 mass parts of grapeseed extract, which further enhances anti-inflammatory properties of the oral cavity hygiene composition.

An oral cavity hygiene composition, in the broadest sense, shall mean composition comprising the above-said mineral-enzyme complex for tooth enamel strengthening as an active ingredient in an effective quantity and an appropriate medium.

An effective mineral-enzyme complex quantity may be different depending on the type of composition, particular sets of ingredients or a combination of substances in the complex etc. Such effective quantity in any individual case may be determined by a professional versed in the field by means of ordinary experiments.

For certain embodiments of the invention (toothpaste) such quantity has been identified by us and cited in the claims of the invention.

An appropriate medium shall mean such that enables to obtain a desired oral cavity hygiene composition in the form of e.g. toothpaste, rinse liquid, tooth powder, gel, foaming rinse, chewing gum etc. In that sense, a composition under the present invention may be represented by a liquid, i.e. a solution of ingredients, e.g. a mouth rinsing liquid; or it may be semi-hard, as for example toothpaste or tooth cleaning gel; or it may be hard, e.g. chewing gum.

If the composition hereunder is a liquid, e.g. a mouth rinsing liquid, then an appropriate medium is represented, as a rule, by a water-glycerin mixture (traditional rinse) or glycerin (high sensitivity dental rinse or applications). Solubilizers and other useful additives may be added to the rinse composition. In particular, the base of the foaming rinse, beside solubilizers, additionally contains surfactants and a foam stabilizing agent.

If the composition is a chewing gum, then an appropriate medium comprises synthetic or natural polymers with plastifiers, flavoring agents, preservatives, sweeteners and other additives.

If the composition is a toothpaste, then an appropriate medium shall comprise solvents, thickeners, surfactants, abrasive substances, emulsifiers, solubilizers, moisturizers, sweeteners, flavoring agents, preservatives and mixtures thereof.

An analysis of prior art shows that for a toothpaste, the abrasive content fluctuates from about 5 to about 60 mass %, which corresponds to the abrasive content of the claimed toothpaste. Abrasive substances prefereable for use in the present invention include silicium dioxide based materials, represented in the invention examples below by Sorbosil (by PQ Corporation) and Tixosil (by Rhodia). However, that list of abrasives is not exhaustive, it being possible to use such substances as abrasive agents as aluminum oxide, calcium carbonate, sodium metaphosphate, potassium metaphosphate, tricalcium phosphate, dehydrated dicalcium phosphate, aluminum silicate, calcined aluminum oxide, bentonite or other silicium based materials or combinations thereof.

An appropriate medium applicable for the obtaining of the composition hereunder in the form of a paste or foaming rinse may comprise a moisturizer. A moisturizer is preferably represented by sorbitol, glycerin and/or polyethylene glycol PEG 400; however, other moisturizers and mixtures thereof with a molecular mass in the range of 200-1000 may also be used. In prior art technical solutions, moisturizer concentrations are usually in the range of from about 0.5 to about 70% of the composition mass.

As a rule, thickeners are present in oral hygiene compositions in quantitites of up to 10 mass %. Thickeners include natural and synthetic resins and colloids. In the present invention, the thickener is represented by sodium carboxy-methylcellulose; besides, the thickener functions are also performed by the above discussed silicium dioxide.

Any of the above listed compositions may additionally contain any appropriate flavoring or sweetening substances.

Toothpastes may additionally comprise a variety of other substances including preservatives, such as methylparaben, sodium methylparaben and chlorophyllic compounds. Such auxiliary substances shall be introduced into compositions hereunder in quantities that do not have an adverse affect on the desired properties and characteristics.

The introduction of emulsion stabilizers in the paste ensures a dispersed state of fats and oils in water emulsions.

Requirements to stabilizers are as follows: ensuring composition stability, inertness to other composition components, no irritating action, non-toxicity, no offensive odor. All of the above requirements are satisfied by Polydon-A, tetrasodium glutamate diacetate and disodium EDTA (Trilon BD), used in particular embodiments of the invention.

A toothpaste may comprise surfactants and, in particular, anionic surfactants, such as sodium lauryl sulphate, sodium myristoyl sarcosinate, cocamidopropyl betaine and sodium lauryl sarcosinate, which possess a multi-functional action: solubilizing, dispersing and moisturizing. Moreover, their function is to form emulsions with other components of oral cavity hygiene compositions, including those with flavoring additives. Beside the above-listed surfactants, other anionic surfactants may also be used, as well as cationic, non-ionic, amphoteric and zwitter-ionic surfactants, or mixtures thereof.

The effect of a surfactant consists in its contribution to the foaming of a toothpaste. The introduction of surfactant with a high degree of foaming contributes to the sensation of effective oral cavity cleaning.

Compositions for oral cavity hygiene according to the present invention may be obtained by mixing the ingredients.

Table 1 provides toothpaste compositions using the claimed mineral-enzyme complex.

Tables 2-4 provide compositions of a variety of rinses using the claimed mineral-enzyme complex.

Table 5 shows the composition of a chewing gum using the claimed mineral-enzyme complex.

Example 1.

To produce toothpaste, complexes of different compositions were used as a mineral-enzyme complex (see footnotes to tables 1-5).

The toothpaste of compositions shown in table 1 was produced as follows: a moisturizer, e.g. glycerin, sorbitol, or polyethylene glycol, was dispersed in water under mixing in a conventional mixer. To the dispersion were added thickeners, a sweetener, a preservative, any active salts and foam stabilizers. To the gel phase were added colorants and a pigment, such as $TiO_2$. If the paste was not gel-like, then any acid or base needed to control pH was added. Those ingredients were mixed to obtain a homogenious phase. Then the mixture was transferred to a high-speed vacuum mixer, wherein an inorganic silicium dioxide based thickener was added to the mixture, and then, in sequence, a silicium dioxide based abrasive along with other abrasive substances to be used in the composition, and finally the mineral-enzyme complex, were added. The mineral-enzyme complex was, prior to introduction, a transparent colorless liquid with white suspension or residue, possessing a specific faint taste and odor, and having a pH of a 5% solution of 7.0-8.0.

Also flavoring agents and surfactants were added, with any component insoluble in water introduced with a moisturizer. The obtained product was in each case a homogeneous semi-hard paste or gel.

The pastes obtained were tested according to the procedure described below.

In the course of control check-ups, the condition of oral cavity hygiene, hard dental tissues, periodontal tissues and oral mucosa was determined according to the criteria described below.

**Research material and methods.**

Tests were conducted with composition C of table 1 and experimental toothpaste composition D with calcium hydroxyapatite (~ 10 mass %) but without tannase.

Trial use of the claimed toothpaste was conducted by individuals with high tooth sensitivity. Altogether, 480 teeth with hypersensitivity were identified in test objects, 6 hypersensitive teeth per test object, on average. Depending on the paste version, test objects were divided into 4 groups:

1 group - composition C toothpaste
2 group - composition D toothpaste

The trial conducted was a double-blind randomized one.

The toothpastes were used by the test objects by themselves twice a day (in the morning and at night), for the period of one month. After the primary check-up, follow-up checks were conducted once a week, for the period of four weeks.

1.1. Study of the cleaning action and cleaning effect

To determine cleaning action, the oral hygiene (Greene and Vermillion) index was used; in the process, PARO disclosing tablets were used.

The cleaning effect was determined by oral hygiene (Greene and Vermillion) index data according to the following formula:

$$\text{Effect } (\%) = [100 \text{ x } (\text{OHI}_0 - \text{OHI}_n)] / \text{OHI}_0$$

where $\text{OHI}_0$ is index value at research start, prior to hygienic procedure;

$\text{OHI}_n$ is index value after n weeks of research, at the last check-up prior to hygienic procedure.

Table 6 shows data on the oral hygiene index dynamics during the four-week period.

1.2. Sensitivity study: tactile and thermal tests

The degree of hyperesthesia severity in gum recession areas prior to and at different times during the use of the toothpaste was determined by diagnostic tests:

1. Probing:

a) cotton swab (CS) tactile sensitivity test;
b) linear probe movement over the tooth surface (LPM).

2. Thermometry:

a) rinsing with a water stream (WSBC);
b) processing with a direct air stream (DAS);
c) processing with a lateral air stream (LAS).

Table 7 shows data on sensitivity dynamics based on diagnostic tests.

1.3. Study of desensitizing action and desensitizing effect

The tooth sensitivity index by L.Yu. Orekhova and S.B. Ulitovsky (TS Orekhova-Ulitovsky index) is determined according to parameters shown in table 4. That index makes it possible to trace teeth condition changes under the influence of desensitizing agents used.

Thus, the TS Orekhova-Ulitovsky index is a sum of values of all described criteria divided by the number of the criteria and multiplied by 100.

$$\frac{\sum (a_1 + ... + a_n)}{5n} \times 100$$

TS Orekhova-Ulitovsky index =

where $\sum$ is the sum of criteria quantitative values;

a1 - number of points for the first criterion;

an - number of points for the n-th criterion;

n - number of criteria used;

5 - number of assessed parameters inside each criterion.

In out problem, the number of criteria and parameters is stable being equal to 11 and 55, respectively. Thus, the formula will look as follows:

$$\frac{\sum (a_1 + ... + a_{11})}{55} \times 100$$

TS Orekhova-Ulitovsky index =

In the denominator, the sum of points for criteria fluctuates in the range of $11 \leq (a1+... a11) \leq 55$, and the index boundaries are $20 \leq$ TS Orekhova-Ulitovsky index $\leq 100$.

Assessment criteria:

81 - 100% - very severe condition;

61 - 80% - severe condition;

between 41 and 60% - relatively compensated tooth sensitivity;

between 21 and 40% - compensated condition against the background of the existing light compensated tooth sensitivity condition;

20% - the tooth (teeth group) healthy with a normal natural sensitivity to external irritants.

In order to study tooth sensitivity for a long time in dynamics, especially under the influence of medication or locally applied oral hygiene aids, we use the Tooth Sensitivity Effectiveness calculation formula (TS Effectiveness):

$$\text{TS Effectiveness (\%)} = [(I1 - In) \times 100] / I1$$

where

I1 is Orekhova-Ulitovsky TS index value determined during the first visit;

In is Orekhova-Ulitovsky TS index value determined during the n-th visit.

Table 8 shows data related to desensitization effectiveness dynamics based on Orekhova-Ulitovsky TS index in the period when the toothpastes were used.

1.4. Study of whitening action and whitening effectiveness

The whitening effect was determined prior to and at different times of toothpaste use by the VITAPAN scale.

After determining the initial values by the VITAPAN scale, correctness of hygienic treatment (tooth cleaning methods and regimen compliance) was examined; appropriate tutoring was conducted as needed.

Table 9 shows data on whitening action dynamics in the period of toothpaste research by the VITAPAN scale.

1.5. Study of remineralizing action and remineralizing effectiveness

The study of remineralizing action was conducted with the help of the TER-test (test of enamel resistance) according to Okushko. Remineralizing effectiveness was calculated on the basis thereof.

Table 10 shows data on remineralizing effectiveness dynamics based on TER-test.

1.6. Study of anti-inflammatory action and anti-inflammatory effectiveness

To determine the periodontal tissue condition, PMA indices were used.

Anti-inflammatory effectiveness was determined on the basis of the obtained values of PMA indices, said effectiveness testifying to the nature of changes in the inflammatory process of the periodontium.

Anti-inflammatory effectiveness was determined according to the following formula:

$$\text{Anti-inflammatory effectiveness (\%)} = [100 \times (PMA_0 - PMA_n)] / PMA_0,$$

where

PMA$_0$ is index value prior to research start;
PMA$_n$ is index value on expiration of n weeks of research, during the final examination.

Table 11 shows data on toothpaste anti-inflammatory effectiveness dynamics based on PMA index.

1.7. Study of blood stopping action and blood stopping effectiveness

To determine the periodontal tissue condition, Muhlemann and Mazor's bleeding index was used.

Based on the obtained bleeding index results, blood-stopping effectiveness was determined testifying to the nature of changes in the inflammatory process of the periodontium. Blood-stopping effectiveness was determined by the formula:

$$\text{Blood-stopping effectiveness (\%)} = [100 \times (BI_0 - BI_n)] / BI_{,0}$$

where BI$_0$ is index value prior to research start;
BI$_n$ is index value on expiration of n weeks of research, during the final examination.

Table 12 shows data on blood-stopping effectiveness.

The obtained results allow to make the conclusion that group 1 that used the claimed composition (composition C) for teeth cleaning demonstrated cleaning effect (41.8%), desensitizing effectiveness (22.1%), whitening effectiveness (42.86%), remineralizing effectiveness (27.3%), anti-inflammatory effectiveness (42.1%), and blood-stopping effectiveness (26.1%).

When the control composition D was used, the majority of indicators were lower, except certain individual indicators (desensitizing and remineralizing effectiveness) that coincided.

Table 1 Composition of toothpastes containing the claimed mineral-enzyme complex.

| Ser. # | Component | Toothpaste compositions, mass % | | | | | Function |
|---|---|---|---|---|---|---|---|
| | | Composition A[1] | Composition B[2] | Composition C[3] | Composition D[4] | Content range | |
| 1. | Mineral-enzyme complex | 6.06 | 1.52 | 10.05 | 11.10 | 1.50-30.00 | Source of mineral substances and tannase enzyme; contributes to strengthening and whitening of enamel, reduction of tooth sensitivity, possesses anti-inflammatory and blood-stopping properties |
| 2. | Sorbitol 70% | 30.00 | 20.00 | 20.00 | 20.0 | 1.00-60.00 | Moisturizer. Influences the product texture imparting special softness and plasticity thereto. |
| 3. | Glycerin (99.7%) | 10.00 | 5.00 | 5.00 | 5.00 | 0.50-70.00 | Moisturizer. Contributes to obtaining a plastic, thixotropic mass, stabilizes foam, improves toothpaste flavor. |
| 4. | Polyethylene glycol PEG 400 | - | 5.00 | 5.00 | - | 0.10-20.00 | Moisturizer. Influences the product texture imparting special softness and plasticity thereto. |
| 5. | Silicium dioxide | 10.0- | 16.0 | 10.0 | 15 | 1.00-60.00 | Thickener and/or abrasive |
| 6. | Sodium carboxy-methylcellulose | 1.00 | 1.5 | 1.50 | 1.50 | 0.5-10.00 | Thickener, structure-forming agent |
| 7. | Xanthan gum | 0.10 | - | - | - | 0.02-3 | Thickener, structure-forming agent |
| 8. | Sodium myristoyl sarcosinate | 4.00 | - | - | - | 2.00-5.00 | Surfactant |
| 9. | Cocamidopropyl betaine | - | 1.00 | - | - | 0.01-10.00 | Surfactant |
| 10. | Sodium lauryl sarcosinate | - | 1.00 | 3.0 | - | 0.01-10.00 | Surfactant |
| 11. | Sodium lauryl sulphate (sodium coco-sulphate) | - | - | - | 2.0 | 0.01-10.00 | Surfactant |
| 12. | Stevia extract | 0.20 | - | 0.20 | 0.20 | 0.05-3 | Sweetener |
| 13. | Xylitol | - | 0.50 | 1.0 | - | 0.01-1 | Sweetener |

EP 2 902 008 B1

(continued)

| Ser. # | Component | Toothpaste compositions, mass % | | | | | Function |
|---|---|---|---|---|---|---|---|
| | | Composition A[1] | Composition B[2] | Composition C[3] | Composition D[4] | Content range | |
| 14. | Sucralose | | 0.05 | | | 0.01-1 | Sweetener |
| 15. | Flavoring agent | 0.50 | 1.0 | 1.0 | 1.0 | 0.05-3 | Flavoring additive |
| 16. | Sodium methylparaben | 0.30 | 0.25 | | 0.300 | 0.05-0.3 | Preservative |
| 17. | Titanium dioxide | | 0.10 | 0.10 | 0.10 | 0.01-5.00 | Whitening agent, colorant |
| 18. | Anise camphor | 0.150 | - | - | - | 0.01-1 | Polyphenol, aseptic |
| 19. | Eucalyptol | 0.10 | - | - | - | 0.01-1 | Polyphenol, aseptic |
| 20. | Thymol | 0.08 | - | - | - | 0.01-1 | Polyphenol, aseptic |
| 21. | Isopropyl methylphenol | 0.100 | - | 0.10 | - | 0.01-0.2 | Aseptic, caries prevention |
| 22. | Sodium monofluorophosphate | - | - | 0.50 | - | 0.01-10.00 | Anti-caries agent |
| 23. | Aminefluoride | - | - | 1.00 | - | 0.01-10.00 | Anti-caries agent |
| 24. | Vitamin E | 0.10 | - | - | 0.10 | 0.01-5.00 | Anti-oxidizing agent, anti-inflammatory action |
| 25. | Tetrasodium glutamate diacetate | 0.50 | - | - | | 0.01-5.00 | Foam stabilizer, chelating and whitening agent |
| 26. | Polydon A | - | - | 0.90 | - | 0.01-5.00 | Foam stabilizer, whitening agent |
| 27. | Trilon BD (disodium EDTA) | - | 0.05 | 0.05 | - | 0.01-5.00 | Foam stabilizer, chelating and whitening agent |
| 28. | Calcium lactate | - | - | - | 1.00 | 0.01-10.00 | Mineralizing agent |
| 29. | Citric acid | 0.10 | 0.20 | - | - | 0.0001-0.50 | Acidity regulating agent |
| 30. | Dry skullcap extract | - | 0.10 | - | - | 0.01-10.00 | Anti-inflammatory, binding, blood-stopping agent |
| 31. | Dry bergenia extract | - | 0.10 | - | - | 0.01-10.00 | Anti-inflammatory, binding, blood-stopping agent |
| 32. | Neem extract | - | 0.05 | - | - | 0.005-5.00 | Antibacterial agent |

(continued)

| Ser. # | Component | Toothpaste compositions, mass % | | | | | Function |
|---|---|---|---|---|---|---|---|
| | | Composition A[1] | Composition B[2] | Composition C[3] | Composition D[4] | Content range | |
| 33. | Water | Up to 100% | | | | | Solvent |

[1] In the mineral-enzyme complex, 0.2 mass parts of tannase and 0.8 mass parts of grapeseed extract are taken per 100 mass parts of hydroxyapatite.
[2] In the mineral-enzyme complex, 2 mass parts of tannase and 50 mass parts of grapeseed extract are taken per 100 mass parts of hydroxyapatite.
[3] In the mineral-enzyme complex, 0.5 mass parts of tannase are taken per 100 mass parts of hydroxyapatite.
[4] In the mineral-enzyme complex, 2 mass parts of tannase and 100 mass parts of grapeseed extract are taken per 100 mass parts of hydroxyapatite.

Table 2 Rinse composition

| Ser. # | Composition component | Composition, mass % | Function |
|---|---|---|---|
| 1 | Mineral-enzyme complex[5] | 1.60 | Contributes to strengthening and whitening of enamel, reduction of tooth sensitivity, possesses anti-inflammatory and blood-stopping properties |
| 2 | Glycerin (99.7%) | 5.00 | Moisturizer, solvent |
| 3 | Trilon BD (disodium EDTA) | 0.05 | Stabilizer, chelating and whitening agent |
| 4 | Sucralose | 0.05 | Sweetener |
| 5 | Sodium methylparaben | 0.30 | Preservative |
| 7 | Xanthan gum | 0.50 | Thickener, structure-forming agent |
| 8 | Calcium lactate | 1.00 | Mineralizing agent |
| 9 | Polyethylene glycol PEG 400 | 5.00 | Moisturizer. |
| 10 | Thymol 001-017 | 0.08 | Polyphenol, aseptic |
| 11 | PEG-40 hydrogenized castor oil | 1.00 | Moisturizer, solubilizer |
| 13 | Vitamin E | 0.05 | Anti-oxidizing agent, anti-inflammatory action |
| 14 | Flavoring agent | 0.30 | Flavoring additive |
| 15 | Anise camphor | 0.10 | Polyphenol, aseptic |
| 16 | Eucalyptol | 0.05 | Polyphenol, aseptic |
| 18 | Citric acid (dry) | 0.20 | Acidity regulating agent |
| 19 | Purified water | Up to 100% | Solvent |

[5] In the mineral-enzyme complex, 10 mass parts of tannase and 50 mass parts of grapeseed extract are taken per 100 mass parts of hydroxyapatite.

Table 3. Composition for rinsing and application for highly sensitive teeth

| Ser. # | Composition component | Composition, mass % | Function |
|---|---|---|---|
| 1 | Mineral-enzyme complex[6] | 20.15 | Source of mineral substances and tannase en zyme; contributes to strengthening and whiten ing of enamel, reduction of tooth sensitivity, possesses anti-inflammatory and blood stopping properties |
| 2 | Glycerin (99.7%) | Up to 100% | Moisturizer. Contributes to obtaining a plastic, thixotropic mass, stabilizes foam, improves toothpaste flavor. |
| 3 | Japanese honeysuckle extract | 1.00 | Antibacterial agent |

(continued)

| Ser. # | Composition component | Composition, mass % | Function |
|---|---|---|---|
| 4 | p-methoxybenzoic acid | 0.10 | Antibacterial agent |
| 5 | Glyceryl monocaprilate (Cosphaderm GMCY) | 0.10 | Antibacterial agent |

[6] In the mineral-enzyme complex, 0.25 mass parts of tannase and 0.5 mass parts of grapeseed extract are taken per 100 mass parts of hydroxyapatite.

Table 4. Foaming rinse composition

| Ser. # | Composition component | Composition, mass % | Function |
|---|---|---|---|
| 1. | Mineral-enzyme complex [7] | 0.71 | Source of mineral substances and tannase enzyme; contributes to strengthening and whitening of enamel, reduction of tooth sensitivity, possesses anti-inflammatory and blood-stopping properties |
| 2. | Sorbitol 70% | 10.00 | Moisturizer. Influences the product texture imparting special softness and plasticity thereto. |
| 3. | Trilon BD (disodium EDTA) | 0.10 | Stabilizer, chelating and whitening agent |
| 4. | Sodium methylparaben | 0.25 | Preservative |
| 5. | L-arginine | 0.100 | Acidity regulating agent |
| 6. | Sucralose | 0.05 | Sweetener |
| 7. | Liquorice extract | 0.05 | Anti-inflammatory and anti-caries agent |
| 8. | Sodium lauryl sarcosinate | 4.00 | Surfactant |
| 9. | Polyvinyl pyrroli-done/vinylacetate | 1.00 | Whitening agent, foam stabilizer |
| 10. | Flavoring agent | 1.00 | Flavoring additive |
| 11. | PEG-40 | 1.00 | Moisturizer, solubilizer |
| 12. | polysorbate - 20 | 1.00 | Moisturizer, solubilizer |
| 13. | carragheenan | 0.05 | Foam stabilizer |
| 14. | Citric acid | 0.20 | Acidity regulating agent |
| 15. | Enzyme complex: lactoferrin, lactoperoxidase, glucose oxidase, potassium thiocyanate, glucose pentaacetate | 0.50 | Antibacterial agent |
| 16. | Purified water | Up to 100% | Solvent |

[7] In the mineral-enzyme complex, 0.25 mass parts of tannase and 0.5 mass parts of grapeseed extract are taken per 100 mass parts of hydroxyapatite.

Table 5. Chewing gum composition.

| Ser. # | Composition component | Composition, mass % | Function |
|---|---|---|---|
| 1 | Mineral-enzyme complex[8] | 2.11 | Source of mineral substances and tannase enzyme; contributes to strengthening and whitening of enamel, reduction of tooth sensitivity, possesses anti-inflammatory and blood-stopping properties |
| 2 | Elastic/rubber base | Up to 100% | Base |
| 3 | Xylitol | 10.000 | Sweetener, anti-caries agent |
| 4 | Flavoring agent | 0.200 | Flavoring additive |
| 5 | Glycerin (99.7%) | 2.000 | Moisturizer. |
| [8] In the mineral-enzyme complex, 5 mass parts of tannase and 0.5 mass parts of grapeseed extract are taken per 100 mass parts of hydroxyapatite. | | | |

Table 6 Oral hygiene (Greene-Vermillion) index dynamics in the period of four weeks

| Ser.# | Distribution by group | Examination period | | | | |
|---|---|---|---|---|---|---|
| | | Start | Week 1 | Week 2 | Week 3 | Week 4 |
| 1 | Composition C | 3.80 | 3.32 | 2.93 | 2.57 | 2.21 |
| 2 | Composition D | 3.69 | 3.28 | 2.89 | 2.49 | 2.26 |

Table 7 Sensitivity dynamics on the basis of diagnostic tests

| Diagnostic tests | In 1 week | | In 2 weeks | | In 3 weeks | | In 4 weeks | |
|---|---|---|---|---|---|---|---|---|
| Compositions | C | D | C | D | C | D | C | D |
| LPM | 8.9 | 10.0 | 17.1 | 16.1 | 22.9 | 25.8 | 31.4 | 32.3 |
| CS | 10.3 | 12.0 | 13.8 | 16.0 | 24.1 | 28.0 | 34.5 | 36.0 |
| WS | 8.8 | 9.5 | 11.6 | 13.6 | 20.4 | 21.1 | 30.4 | 32.8 |
| LAS | 9.3 | 9.6 | 14.1 | 15.0 | 18.4 | 23.6 | 32.3 | 33.4 |
| DAS | 10.6 | 10.8 | 18.8 | 19.0 | 22.4 | 24.5 | 32.8 | 33.8 |

Table 8 Desensitizing effectiveness dynamics by Orekhova-Ulitovsky TS index in the period of toothpaste use

| Distribution by group | Examination period | | | |
|---|---|---|---|---|
| | Week 1 | Week 2 | Week 3 | Week 4 |
| Composition C | 6.8% | 11.4% | 16.5% | 22.1% |
| Composition D | 7.0% | 11.7% | 17.9% | 22.0% |

Table 9 Whitening action dynamics in the period of toothpaste examination by VITAPAN scale

| Distribution by group | Examination period | | | | |
|---|---|---|---|---|---|
| | Initial | In 1 week | In 2 weeks | In 3 weeks | In 4 weeks |
| Composition C | A3.5 | A3.5 | A3 | A2 | A2 |
| Composition D | A3.5 | A3.5 | A3.5 | A3 | A2.5 |

Table 10 Remineralizing effectiveness dynamics by TER-test

| Distribution by group | Examination period | | | |
|---|---|---|---|---|
| | Week 1 | Week 2 | Week 3 | Week 4 |
| Composition C | 9.1% | 18.2% | 27.3% | 27.3% |
| Composition D | 10.0% | 20.0% | 30.0% | 30.0% |

Table 11 Toothpaste anti-inflammatory effectiveness dynamics by PMA index

| Distribution by group | Examination period | | | |
|---|---|---|---|---|
| | Week 1 | Week 2 | Week 3 | Week 4 |
| Composition C | 7.1% | 14.9% | 23.6% | 42.1% |
| Composition D | 5.8% | 10.5% | 21.1% | 38.9% |

Table 12 Blood-stopping effectiveness

| Distribution by group | Examination period | | | |
|---|---|---|---|---|
| | Week 1 | Week 2 | Week 3 | Week 4 |
| Composition C | 8.3% | 13.0% | 20.9% | 26.1% |
| Composition D | 5.5% | 8.5% | 11.0% | 14.5% |

**Claims**

1. Mineral-enzyme complex for tooth enamel strengthening and whitening, wherein said complex contains calcium hydroxyapatite and tannase with the following component proportions: from 0.2 to 10 parts of tannase per 100 mass parts of calcium hydroxyapatite.

2. Complex according to claim 1, wherein it additionally contains grapeseed extract with the following component proportions: from 0.2 to 10 parts of tannase and up to 100 mass parts of grapeseed extract per 100 mass parts of calcium hydroxyapatite.

3. Composition for oral cavity hygiene, wherein it contains a mineral - enzyme complex for tooth enamel strengthening and whitening according to any of claims 1 and 2 in an effective quantity and an appropriate medium.

4. Composition according to claim 3, wherein it is represented by a toothpaste.

5. Composition according to claim 3 wherein it is represented by a rinsing composition.

6. Composition according to claim 3, wherein it is represented by a foaming rinsing composition.

7. Composition according to claim 3, wherein it is represented by a chewing gum.

**8.** Toothpaste for oral cavity hygiene, wherein it contains a mineral - enzyme complex for tooth enamel strengthening and whitening according to any of claims 1 and 2 in an effective quantity, and an appropriate medium comprising substances selected from the group consisting of abrasive substances, moisturizers, thickeners, surfactants and solvents.

**9.** Toothpaste according to claim 8, wherein it contains a medium comprising water as a solvent, silicium dioxide as an abrasive substance, sodium carboxy-methylcellulose as a thickener, at least one moisturizer selected from the group consisting of glycerin, sorbitol and polyethylene glycol, and sodium lauryl sarcosinate as a surfactant, with the following component proportions, mass %:

| | |
|---|---|
| Mineral-enzyme complex | 1.50 - 30.00 |
| Silicium dioxide | 1.00 - 60.00 |
| Sodium carboxy-methylcellulose | 0.50 - 10.00 |
| Moisturizer | 0.50 - 70.00 |
| Sodium lauryl sarcosinate | 0.01 - 10.00 |
| Water | the rest. |

**10.** Toothpaste according to claim 8, wherein it additionally contains xanthan gum as a thickener in a quantity not exceeding 3 mass %.

**11.** Toothpaste according to claim 8, wherein it additionally contains substances selected from the group consisting of foam stabilizers, colorants, preservatives, flavoring agents, anti-oxidizing agents, mineralizing agents, anti-inflammatory binding agents, aseptics, anti-caries agents and mixtures thereof with the following component proportions, mass %:

| | |
|---|---|
| Foam stabilizers | max. 5.00 |
| Colorants | max. 5.00 |
| Preservatives | max. 0.30 |
| Flavoring agents | max. 3.00 |
| Anti-oxidizing agents | max. 5.00 |
| Mineralizing agents | max. 10.00 |
| Anti-inflammatory binding agents | max. 10.00 |
| Aseptics | max. 1.00 |
| Anti-caries agents | max. 10.00 |

**Patentansprüche**

**1.** Mineral-Enzym-Komplex zur Stärkung und Aufhellung des Zahnschmelzes, wobei besagter Komplex Calcium-Hydroxylapatit und Tannase mit den folgenden Komponentenanteilen enthält: von 0,2 bis 10 Teilen Tannase pro 100 Masseteile Calcium-Hydroxylapatit.

**2.** Komplex nach Anspruch 1, wobei er zusätzlich Traubenkernextrakt mit den folgenden Komponentenanteilen enthält: von 0,2 bis 10 Teilen Tannase und bis zu 100 Masseteilen Traubenkernextrakt pro 100 Masseteile Calcium-Hydroxylapatit.

**3.** Zusammensetzung zur Mundhöhlenhygiene, wobei sie einen Mineral-Enzym-Komplex zur Stärkung und Aufhellung des Zahnschmelzes nach einem der Ansprüche 1 und 2 in einer wirksamen Menge und ein geeignetes Medium enthält.

**4.** Zusammensetzung nach Anspruch 3, wobei sie durch eine Zahnpasta repräsentiert wird.

**5.** Zusammensetzung nach Anspruch 3, wobei sie durch eine Zusammensetzung zum Spülen repräsentiert wird.

**6.** Zusammensetzung nach Anspruch 3, wobei sie durch eine schäumende Zusammensetzung zum Spülen repräsentiert wird.

**7.** Zusammensetzung nach Anspruch 3, wobei sie durch einen Kaugummi repräsentiert wird.

**8.** Zahnpasta für die Hygiene der Mundhöhle, wobei sie einen Mineral-Enzym-Komplex zur Stärkung und Aufhellung des Zahnschmelzes nach einem der Ansprüche 1 und 2 in einer wirksamen Menge und ein geeignetes Medium enthält, das Substanzen umfasst, die ausgewählt sind aus der Gruppe bestehend aus Schleifmitteln, Feuchtigkeitsspendern, Verdickungsmitteln, Tensiden und Lösungsmitteln.

**9.** Zahnpasta nach Anspruch 8, wobei sie ein Medium enthält, das Wasser als Lösungsmittel, Siliziumdioxid als Schleifmittel, Natrium-Carboxy-Methylcellulose als Verdickungsmittel, mindestens einen Feuchtigkeitsspender, ausgewählt aus der Gruppe bestehend aus Glycerin, Sorbit und Polyethylenglykol, und Natriumlaurylsarcosinat als Tensid mit den folgenden Komponentenanteilen, in Masse-%, enthält:

| | |
|---|---|
| Mineral-Enzym-Komplex | 1,50 - 30,00 |
| Siliciumdioxid | 1,00 - 60,00 |
| Natrium-Carboxy-Methylcellulose | 0,50 - 10,00 |
| Feuchtigkeitsspender | 0,50 - 70,00 |
| Natriumlaurylsarcosinat | 0,01 - 10,00 |
| Wasser | der Rest. |

**10.** Zahnpasta nach Anspruch 8, wobei sie zusätzlich XanthanGummi als Verdickungsmittel in einer Menge von höchstens 3 Masse-% enthält.

**11.** Zahnpasta nach Anspruch 8, wobei sie zusätzlich Substanzen enthält, ausgewählt aus der Gruppe bestehend aus Schaumstabilisatoren, Farbstoffen, Konservierungsmitteln, Aromastoffen, Antioxidationsmitteln, Mineralisierungsmitteln, entzündungshemmenden Bindemitteln, Aseptika, Anti-Kariesmitteln und Mischungen derselben mit den folgenden Komponentenanteilen, in Masse-%:

| | |
|---|---|
| Schaumstabilisatoren | max. 5,00 |
| Farbstoffe | max. 5,00 |
| Konservierungsmittel | max. 0,30 |
| Aromastoffe | max. 3,00 |
| Anti-Oxidationsmittel | max. 5,00 |
| Mineralisierungsmittel | max. 10,00 |
| entzündungshemmende Bindemittel | max. 10,00 |
| Aseptika | max. 1,00 |
| Anti-Kariesmittel | max. 10,00 |

**Revendications**

**1.** Complexe minéral-enzyme destiné au renforcement et au blanchiment de l'émail dentaire, **caractérisé en ce que** ledit complexe contient de l'hydroxyapatite de calcium et de la tannase selon les proportions de composants suivantes : de 0,2 à 10 parties de tannase pour 100 parties en masse d'hydroxyapatite de calcium.

**2.** Complexe selon la revendication 1, **caractérisé en ce qu'**il contient en outre un extrait de pépins de raisin selon les proportions de composants suivantes : de 0,2 à 10 parties de tannase et jusqu'à 100 parties en masse d'extrait de pépins de raisin pour 100 parties en masse d'hydroxyapatite de calcium.

**3.** Composition destinée à l'hygiène de la cavité orale, **caractérisée en ce qu'**elle contient un complexe minéral-enzyme destiné au renforcement et au blanchiment de l'émail dentaire selon l'une quelconque des revendications 1 et 2, selon une quantité efficace et dans un milieu approprié.

**4.** Composition selon la revendication 3, **caractérisée en ce qu'**elle est représentée par un dentifrice.

**5.** Composition selon la revendication 3, **caractérisée en ce qu'**elle est représentée par une composition de rinçage.

**6.** Composition selon la revendication 3, **caractérisée en ce qu'**elle est représentée par une composition de rinçage moussante.

**7.** Composition selon la revendication 3, **caractérisée en ce qu'**elle est représentée par un chewing-gum.

**8.** Dentifrice destiné à l'hygiène de la cavité orale, **caractérisé en ce qu'**il contient un complexe minéral-enzyme destiné au renforcement et au blanchiment de l'émail dentaire selon l'une quelconque des revendications 1 et 2, selon une quantité efficace, et un milieu approprié comprenant des substances choisies dans le groupe constitué par les substances abrasives, les agents hydratants, les épaississants, les agents tensioactifs et les solvants.

**9.** Dentifrice selon la revendication 8, **caractérisé en ce qu'**il contient un milieu comprenant de l'eau comme solvant, du dioxyde de silicium comme substance abrasive, de la carboxyméthylcellulose de sodium comme épaississant, au moins un agent hydratant choisi dans le groupe constitué par le glycérol, le sorbitol et le polyéthylène glycol, et du laurylsarcosinate de sodium comme agent tensioactif, avec les proportions de composants suivantes, en % en masse :

| | |
|---|---|
| Complexe minéral-enzyme | 1,50-30,00 |
| Dioxyde de silicium | 1,00-60,00 |
| Carboxyméthylcellulose de sodium | 0,50-10,00 |
| Agent hydratant | 0,50-70,00 |
| Laurylsarcosinate de sodium | 0,01-10,00 |
| Eau | qsp. |

**10.** Dentifrice selon la revendication 8, **caractérisé en ce qu'**il contient en outre de la gomme xanthane comme épaississant, selon une quantité n'excédant pas 3% en masse.

**11.** Dentifrice selon la revendication 8, **caractérisé en ce qu'**il contient en outre des substances choisies dans le groupe constitué par les stabilisateurs de mousse, les colorants, les conservateurs, les agents aromatisants, les agents antioxydants, les agents minéralisants, les agents fixants anti-inflammatoires, les agents aseptiques, les agents anti-caries, et des mélanges de ceux-ci, avec les proportions de composants suivantes, en % en masse :

| | |
|---|---|
| Stabilisateurs de mousse | max. 5,00 |
| Colorants | max. 5,00 |
| Conservateurs | max. 0,30 |
| Agents aromatisants | max. 3,00 |
| Agents antioxydants | max. 5,00 |
| Agents minéralisants | max. 10,00 |
| Agents fixants anti-inflammatoires | max. 10,00 |
| Agents aseptiques | max. 1,00 |
| Agents anti-caries | max. 10,00. |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2355380
- RU 2131723
- RU 2060030
- RU 2416391
- RU 2355420

**Non-patent literature cited in the description**

- **A.A. LEONTIEV et al.** Clinical Research of 'Asepta Sensitive' Anti-Sensitive Toothpaste. *Stomatology Today,* 2009, vol. 87 (7